# FASCICULE DE BREVET EUROPEEN

(11) **EP 3 880 365 B1**
(45) Date de publication et mention de la délivrance du brevet: **10.08.2022**
(21) Numéro de dépôt: 19818230.5
(22) Date de dépôt: 07.11.2019
(51) Int. Cl.: B01L 7/00, B01L 3/00, G01N 15/06, G01N 15/14, G01N 21/64, G01N 1/40, G01N 15/00, G01N 1/28, G01N 15/10, C12Q 1/68, G01N 35/00

(54) **SYSTÈME AUTOMATISÉ DE PRÉPARATION, DE DÉTECTION ET D'ANALYSE D'UN ÉCHANTILLON FLUIDIQUE**
AUTOMATISIERTES SYSTEM ZUR VORBEREITUNG, DETEKTION UND ANALYSE EINER FLÜSSIGKEITSPROBE
AUTOMATED SYSTEM FOR PREPARING, DETECTING AND ANALYSING A FLUID SAMPLE

(30) Priorité: 12.11.2018 FR 1860414
(43) Date de publication de la demande: 22.09.2021
(73) Titulaire: Commissariat à l'Énergie Atomique et aux Énergies Alternatives, 75015 Paris (FR); TotalEnergies OneTech, 92400 Courbevoie (FR)
(72) Inventeur: DEN DULK, Remco, 38054 Grenoble cedex 09 (FR); ECHAMPARD, Camille, 38054 Grenoble cedex 09 (FR); CHARLES, Raymond, 38054 Grenoble cedex 09 (FR); ALESSIO, Manuel, 38054 Grenoble cedex (FR); SARRUT-RIO, Nicolas, 38180 Grenoble cedex 09 (FR); BOURDAT, Anne-Gaëlle, 38054 Grenoble cedex 9 (FR); BAQUE, Mélissa, 38054 Grenoble cedex 09 (FR); BOIZOT, François, 38054 Grenoble cedex 09 (FR); MALINGE, Jean, 64230 LESCAR (FR); SOUQUET, Pierre, 64018 Pau Cedex (FR)
(74) Mandataire: INNOV-GROUP
(86) Numéro de dépôt international: PCT/FR2019/052662
(87) Numéro de publication internationale: WO 2020/099763

(56) Documents cités:
- WO-A1-2015/015175
- US-A1- 2011 014 606
- US-A1- 2018 099 276
- Remco Den Dulk: "FlowPad, a generic microfluidics platform for a wide range of applications", 11th EPIZONE Annual Meeting "Crossing Barriers" 19-21 September 2017, 21 septembre 2017 (2017-09-21), XP055608282, Paris, France Extrait de l'Internet: URL:https://www.epizone-eu.net/upload_mm/0 /c/6/37a1e21a-2e43-42d4-ac53-5dcc6cf732e1_ 2109%20KEYNOTE%20AM%20DEN%20DULK.pdf [extrait le 2019-07-24]
- FLAENDER MÉLANIE ET AL: "Grinding Lysis (GL): A microfluidic device for sample enrichment and mechanical lysis in one", SENSORS AND ACTUATORS B: CHEMICAL, ELSEVIER BV, NL, vol. 258, 21 novembre 2017 (2017-11-21), pages 148-155, XP085338438, ISSN: 0925-4005, DOI: 10.1016/J.SNB.2017.11.082
- DAFENG CHEN ET AL: "An integrated, self-contained microfluidic cassette for isolation, amplification, and detection of nucleic acids", BIOMEDICAL MICRODEVICES, KLUWER ACADEMIC PUBLISHERS, BO, vol. 12, no. 4, 17 avril 2010 (2010-04-17) , pages 705-719, XP019814145, ISSN: 1572-8781

## Description

### Domaine technique de l'invention

La présente invention se rapporte à un système automatisé de préparation, de détection et d'analyse d'un échantillon qui comporte un appareil d'analyse complet et une cartouche destinée à être insérée de manière amovible dans l'appareil d'analyse. Le système de préparation, de détection et d'analyse est notamment particulièrement adapté pour détecter la présence de bactéries dans un échantillon fluidique.

### Etat de la technique

Le développement de micro-organismes tels que bactéries, champignons, algues, levures peut avoir des impacts négatifs dans une installation industrielle. Le développement de biofilms peut causer de nombreux dégâts parmi lesquels : corrosion, bouchage, acidification, affectation du produit.

Il s'avère donc nécessaire de réaliser un suivi de la présence des micro-organismes problématiques en milieu industriel, en réalisant des prélèvements réguliers dans les zones sensibles... L'échantillon pourra être prélevé sous la forme d'un biofilm ou sous la forme d'un fluide, par exemple des eaux de production venant d'un réservoir de pétrole.

Les analyses de contrôle et de suivi des microorganismes réalisées sur ces fluides sont souvent basées sur la culture. Classiquement, les échantillons sont inoculés par une gamme de dilutions en série, dans un milieu contenant les nutriments et conditions physico-chimiques optimales pour la croissance des micro-organismes d'intérêt, et incubés à la température idéale. En cas de croissance, l'utilisation d'indicateurs de pH ou la formation de précipités colorés indique la présence de microorganismes. Cette approche présente l'avantage d'être simple, peu coûteuse et surtout elle peut être déployée facilement dans un laboratoire sommaire de contrôle d'installation industrielle. Elle présente cependant de nombreux inconvénients :
- Temps de réponse long dû au temps d'incubation - il peut en effet être de plusieurs jours à plusieurs semaines si les temps de doublement sont importants ;
- Certains microorganismes ne sont pas cultivables dans les conditions expérimentales et ne seront donc pas détectés ;
- Nécessité d'une formation avancée pour appréhender les techniques d'inoculations aseptique et/ou en anaérobiose - Des variations de résultats sont ainsi possibles d'un opérateur à un autre ;
- Nécessité d'un certain savoir-faire pour interpréter les résultats obtenus.

De nouvelles méthodes d'analyse moléculaire basées sur la détection d'ADN se sont développées depuis quelques années. Elles nécessitent cependant du personnel très bien formé aux techniques de la biologie moléculaire, notamment pour ce qui est du pipetage et de l'utilisation des micropipettes. L'extraction de l'ADN est aussi une étape assez longue du processus, impliquant plusieurs étapes de lyse, concentration et purification avant analyse. De telles analyses sont donc généralement réalisées en dehors du site industriel après préservation et transport des échantillons vers des laboratoires spécialisés.

Des solutions d'analyse sont décrites dans la demande de brevet US2011/014606A1 ainsi que dans les publications référencées ci-dessous :
Remco Den Dulk : « flowpad, a generic microfluidics platform for a wide range of applications », 11th EPIZONE Annual meeting « Crossing Barriers » 19-21, September 2017, 21 septembre 2017, XP055608282, Paris, France*.*
Flaender Mélanie et Al : « Grinding lysis (GL) : A microfluidic device for sample enrichment and mechanical lysis in one », Sensors and Actuators : Chemical, Elsevier BV, NL, vol 258, 21 novembre 2017, pages 148-155, XP085338438, ISSN : 0925-4005, DOI:10.1016/J.SNB.2017.11.082*.*

Pour être au plus proche du terrain et permettre une réaction rapide lors de prolifération, ou une optimisation précise des traitements anti-bactériens employés, il existe donc un besoin de disposer d'un système de préparation, de détection et d'analyse :
- qui soit entièrement automatisé de manière à limiter les interventions humaines,
- qui permette de déterminer de manière fiable et rapide la nature et la quantité des espèces biologiques présentes dans un échantillon, même complexe,
- qui ne nécessite pas l'intervention d'un personnel qualifié,
- qui soit facilement transportable et puisse être déployé dans un laboratoire sommaire de site industriel, et
- qui puisse s'adapter aux cibles à détecter.

### Exposé de l'invention

Ce but est atteint par un système automatisé de préparation, de détection et d'analyse d'un premier échantillon fluidique contenant des espèces biologiques, ledit système de préparation, de détection et d'analyse comprenant :
- Au moins une cartouche fluidique qui comporte au moins un module fluidique de concentration et de lyse et un module fluidique de détection comprenant un réseau de plusieurs chambres d'amplification agencées en parallèle par rapport à un canal fluidique de répartition, ledit module fluidique de concentration et de lyse comprenant un dispositif de concentration et de lyse qui comporte une chambre de préparation, une surface d'appui abrasive réalisée dans ladite chambre de préparation, un filtre et une membrane souple et déformable venant refermer ladite chambre,
- Un appareil comprenant :
   o Un châssis doté d'au moins une platine destinée à recevoir ladite cartouche fluidique de manière amovible,
   o Un ensemble mécanique comprenant au moins une tige mobile fixée audit châssis et comprenant une extrémité libre agencée pour coopérer avec ladite membrane souple du module fluidique de concentration et de lyse,
   o Un système pneumatique commandé pour permettre une circulation d'un fluide à travers ladite cartouche,
   o Au moins une unité de chauffage portée par la platine et agencée pour chauffer le réseau de chambres en parallèle lors d'une réaction d'amplification, cyclique ou isotherme,
   o Un système optique de mesure de fluorescence à travers une ou plusieurs des chambres d'amplification du module fluidique de détection de la cartouche,
   o Une unité de commande et de traitement configurée pour mettre en œuvre une séquence d'analyse qui comporte au moins les étapes suivantes :
      ▪ Commande du système pneumatique pour injection du premier échantillon fluidique à travers le filtre du dispositif de concentration et de lyse pour récupérer les espèces biologiques présentes dans le premier échantillon,
      ▪ Commande du système pneumatique pour générer un flux d'air de séchage dans un circuit fluidique du module fluidique de concentration et de lyse, ledit circuit fluidique traversant ledit filtre du dispositif de concentration et de lyse,
      ▪ Commande de l'ensemble mécanique pour actionnement de la tige dans un mouvement d'appui de la membrane souple contre la surface d'appui abrasive afin de lyser les espèces biologiques contenues dans le premier échantillon fluidique,
      ▪ Commande du système pneumatique pour évacuer un deuxième échantillon fluidique en dehors de la chambre de préparation vers ledit canal fluidique de répartition de la cartouche,
      ▪ Commande du système pneumatique pour remplissage en parallèle et de manière simultanée des chambres d'amplification du réseau avec le deuxième échantillon fluidique,
      ▪ Commande de l'unité de chauffage pour chauffer ledit deuxième échantillon fluidique présent dans chaque chambre du réseau,
      ▪ Commande du système optique de mesure pour mesurer la fluorescence dans chaque chambre d'amplification contenant une fraction du deuxième échantillon fluidique,
      ▪ Enregistrement des résultats de mesure de fluorescence,
      ▪ Application d'un algorithme d'analyse sur les résultats de mesure obtenus et génération de données qualitatives et quantitatives des espèces biologiques présentes dans le premier échantillon fluidique.

Selon une particularité, l'unité de commande et de traitement est configurée pour exécuter :
- Un premier module configuré pour envoyer des commandes au dispositif de concentration et de lyse ainsi qu'au système pneumatique,
- Un deuxième module configuré pour envoyer des commandes à l'unité de chauffage, au système pneumatique ainsi qu'au système optique de mesure,
- Un troisième module configuré pour recevoir des images en provenance du système optique de mesure et de traiter les images.

Selon une autre particularité, le système comporte une base de données stockant les résultats d'analyse antérieures et en ce que le troisième module est configuré pour se reporter à ladite base de données afin de générer des courbes de variation de concentration de chaque espèce biologique détecté.

Selon une autre particularité, chaque module fluidique peut comporter des filtres hydrophobes agencés sur des canaux pneumatiques reliés directement au système pneumatique.

Selon une autre particularité, le module fluidique de concentration et de lyse et le module fluidique de détection comporte plusieurs vannes fluidiques commandables par l'unité de commande et de traitement.

Selon une autre particularité, le système comporte une cassette intégrant n cartouches fluidiques, avec n supérieur ou égal à 2.

Selon une autre particularité, le système comporte :
- n ensembles mécaniques comprenant chacun au moins une tige et associée à chaque cartouche,
- n unités de chauffage agencés chacune pour chauffer le réseau de chambres de chaque cartouche,
- n/2 système optique de mesure de fluorescence, configuré pour se déplacer entre deux positions, afin d'imager les n cartouches du système.

L'invention concerne également un procédé de préparation, de détection et d'analyse d'un échantillon fluidique, mis en œuvre à partir du système tel que défini dans l'une des revendications précédentes, ledit procédé comportant les étapes suivantes :
- Commande du système pneumatique pour injection du premier échantillon fluidique à travers le filtre du dispositif de concentration et de lyse pour récupérer les espèces biologiques présentes dans le premier échantillon,
- Commande du système pneumatique pour générer un flux d'air de séchage dans un circuit fluidique du module fluidique de concentration et de lyse, ledit circuit fluidique traversant ledit filtre du dispositif de concentration et de lyse,
- Commande de l'ensemble mécanique pour actionnement de la tige dans un mouvement d'appui de la membrane souple contre la surface d'appui abrasive afin de lyser les espèces biologiques contenues dans le premier échantillon fluidique,
- Commande du système pneumatique pour évacuer un deuxième échantillon fluidique en dehors de la chambre de préparation vers ledit canal fluidique de répartition de la cartouche,
- Commande du système pneumatique pour remplissage en parallèle et de manière simultanée des chambres d'amplification du réseau avec le deuxième échantillon fluidique,
- Commande de l'unité de chauffage pour chauffer ledit deuxième échantillon fluidique présent dans chaque chambre du réseau,
- Commande du système optique de mesure pour mesurer la fluorescence dans chaque chambre d'amplification contenant une fraction du deuxième échantillon fluidique,
- Enregistrement des résultats de mesure de fluorescence,
- Application d'un algorithme d'analyse sur les résultats de mesure obtenus et génération de données qualitatives et quantitatives des espèces biologiques présentes dans le premier échantillon fluidique.

L'invention concerne une utilisation du système tel que défini ci-dessus pour détecter la présence d'espèces biologiques dans un échantillon fluidique injecté dans ladite cartouche fluidique du système.

### Brève description des figures

D'autres caractéristiques et avantages vont apparaître dans la description détaillée qui suit faite en regard des figures annexées listées ci-dessous :
- La figure 1 représente de manière schématique un système de préparation, de détection et d'analyse conforme à l'invention ;
- La figure 2 représente un schéma illustrant le principe de fonctionnement du système de préparation, de détection et d'analyse de l'invention ;
- La figure 3 représente de manière schématique un dispositif de concentration et de lyse pouvant être employé dans le système de préparation, de détection et d'analyse conforme à l'invention ;
- Les figures 4A à 4M représentent les différentes étapes mises en œuvre dans le module fluidique de concentration et de lyse employé dans le système de l'invention ;
- Les figures 5A à 5G représentent les différentes étapes mises en œuvre dans le module fluidique de détection employé dans le système de l'invention ;
- La figure 6 représente une variante de réalisation avantageuse du système de préparation, de détection et d'analyse de l'invention.

### Description détaillée d'au moins un mode de réalisation

L'invention concerne un système automatisé de préparation, de détection et d'analyse d'un échantillon fluidique comprenant des espèces biologiques.

Par espèces biologiques, de manière non limitative, on entend les micro-organismes, les bactéries, les cellules, les spores, les champignons...

L'échantillon à analyser pourra se présenter sous la forme d'un fluide prélevé directement sur site ou être obtenu après dilution d'un biofilm prélevé sur le site. Par fluide, on entend préférentiellement un liquide.

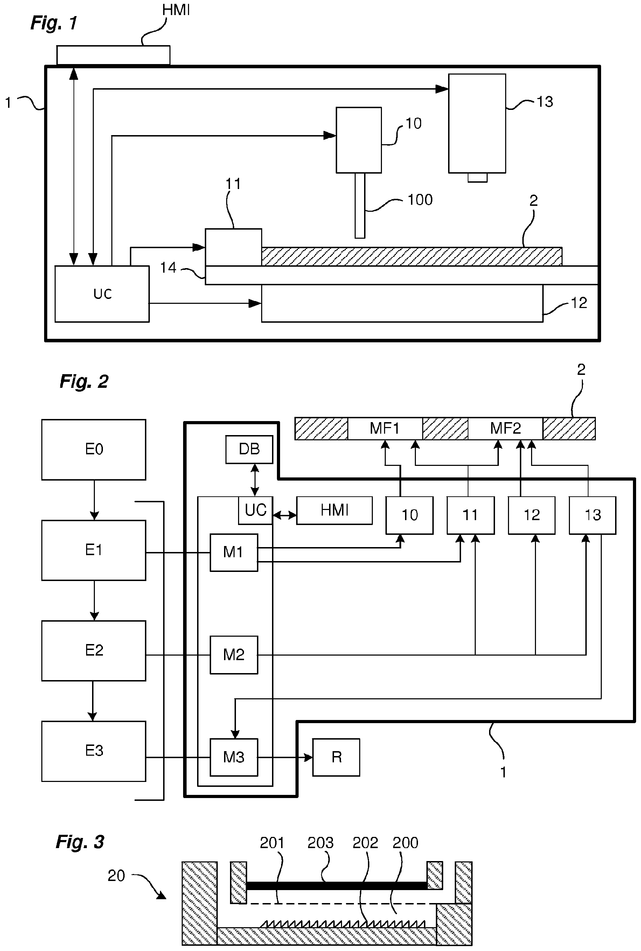

En référence à la figure 2, une analyse d'un échantillon liquide pouvant comporter des espèces biologiques du type de celles décrites ci-dessus, se déroule classiquement en suivant les différentes étapes suivantes :
- Une étape E0 préliminaire de préparation de l'échantillon prélevé. On verra que cette étape n'est pas mise en œuvre par le système de préparation, de détection et d'analyse de l'invention. Il peut s'agir par exemple de diluer le prélèvement si celui-ci s'avère solide. C'est par exemple le cas lorsque le prélèvement est un biofilm. Une fois préparé, l'échantillon se présente sous la forme d'un liquide de volume égal à au moins 1ml.
- Une deuxième étape E1 de préparation consistant en une concentration et une lyse. Lors de cette étape, l'échantillon préparé lors de l'étape E0 est d'abord filtré afin de ne conserver que les espèces biologiques. Ces espèces biologiques sont ensuite rincées et purifiées par un liquide de rinçage adapté. Puis les espèces biologiques sont lysées mécaniquement afin de libérer un matériel biologique à analyser. Un nouveau filtrage peut permettre de sélectionner ce matériel biologique et de le séparer des polluants générés lors de la lyse.
- Une étape E2 de détection, pouvant être mise en œuvre en utilisant une réaction d'amplification de type Q-PCR (pour "Quantitative - Polymerase Chain Reaction") ou de type LAMP, RPA, séquençage NGS (Nanopore). L'échantillon contenant le matériel biologique obtenu après la lyse peut être mélangé avec des réactifs secs. Le mélange est injecté sous forme liquide dans plusieurs chambres d'amplification agencées en parallèle, alimentées par un canal de répartition. Chaque chambre peut contenir un jeu d'amorces distinct selon la cible à détecter. Les chambres sont chauffées pour faire subir à chaque échantillon présent dans une chambre, un ou plusieurs cycles thermiques successifs d'un protocole d'amplification, ou les maintenir à une température isotherme dans le cas d'une détection de type LAMP ou RPA. Des images de fluorescence sont capturées pendant chaque cycle thermique du protocole d'amplification et sont enregistrées.
- Une étape E3 d'analyse des images de fluorescence capturées. Un algorithme construit la courbe d'intensité en fluorescence pour chaque échantillon présent dans une chambre, en tenant compte des cycles thermiques appliqués. La concentration en espèces biologiques ciblées peut ainsi être déterminée pour chaque échantillon analysé.

Selon l'invention, pour mettre en œuvre l'étape E1 de concentration et de lyse et l'étape E2 de détection et l'étape E3 d'analyse, on utilise un système automatisé de préparation, de détection et d'analyse qui comporte un appareil d'analyse 1 et au moins une cartouche 2 fluidique à insérer dans l'appareil 1 pour l'analyse.

Le système présente la particularité que certaines fonctionnalités sont apportées par l'appareil 1 et que d'autres sont apportées directement par la cartouche 2 qui est insérée dans l'appareil. Autrement dit, l'appareil d'analyse et la cartouche doivent coopérer entre eux pour faire fonctionner le système et l'analyse ne peut être mise en œuvre sans l'une ou l'autre de ces deux entités.

La cartouche 2 est réalisée sous la forme d'au moins une carte fluidique (même micro-fluidique). Cette carte peut avoir le format d'une carte de crédit ou d'un autre format. Elle peut être réalisée dans un matériau transparent de type PMMA (Poly-méthacrylate de Méthyle) ou équivalent. La cartouche 2 insérée dans l'appareil présente avantageusement une configuration (notamment fluidique) qui est toujours identique de manière à coopérer avec les éléments de l'appareil qui sont nécessaires à la mise en œuvre de l'analyse.

La cartouche comporte avantageusement deux modules fluidiques distincts :
- Un premier module fluidique MF1 destiné à la concentration et à la lyse des espèces biologiques présentes dans l'échantillon à analyser ;
- Un deuxième module fluidique MF2 dédié à la détection des espèces biologiques séparées par le premier module.

De manière générale et non limitative, le premier module fluidique MF1 de la cartouche 2 peut comporter notamment :
- Un premier point d'injection destiné à recevoir l'échantillon à analyser ;
- Un deuxième point d'injection pour injecter un liquide de rinçage ;
- Un troisième point d'injection pour injecter un liquide d'élution ;
- Un dispositif 20 fluidique de concentration et de lyse des espèces biologiques présentes dans l'échantillon. De manière non limitative, ce dispositif 20 peut présenter une architecture telle que celle représentée sur la figure 3 et déjà décrite dans la demande de brevet EP3222989A1**.** Il peut ainsi comporter une chambre 200 de concentration et de lyse dans laquelle est placée l'échantillon, un filtre 201, une surface d'appui abrasive 202 placée dans la chambre 200 et contre laquelle les espèces biologiques peuvent être broyées et une membrane souple 203 et déformable venant refermer la chambre ; Une autre architecture de dispositif est également décrite dans la demande de brevet WO2015/181743A1**.**
- Un réservoir de stockage de l'échantillon obtenu après concentration et lyse ;
- Un réservoir poubelle ;
- Un circuit fluidique adapté pour joindre les différents éléments du module ;
- Un circuit pneumatique comprenant des entrées/sorties pneumatiques adaptées pour faire circuler le fluide entre les éléments à travers le circuit fluidique.

De manière générale et non limitative, le deuxième module fluidique MF2 de la cartouche 2 peut comporter notamment :
- Un premier point d'injection de l'échantillon à analyser, ce premier point d'injection étant relié par une liaison fluidique au réservoir de stockage du premier module fluidique ;
- Un réseau de plusieurs chambres distinctes alimentées en parallèle par un canal de répartition relié audit premier point d'injection du module d'analyse ;
- Un circuit fluidique adapté pour joindre les différents éléments du module ;
- Un circuit pneumatique comprenant des entrées/sorties pneumatiques adaptées pour permettre la circulation de fluide dans le circuit fluidique du module.

L'appareil d'analyse comporte pour sa part les éléments suivants :
- Une platine 14 formant une surface d'appui plane pour recevoir ladite cartouche 2 fluidique de manière amovible ; Après positionnement sur la platine, la cartouche 2 reste fixe ;
- Un ensemble mécanique 10 composé d'une spatule 100 ou tige mobile, entraînée en rotation par un moteur par rapport à la cartouche 2 ; La spatule 100 est commandée pour venir en appui, par l'extérieur, contre la membrane 203 souple du dispositif 20 fluidique de concentration et de lyse afin d'étirer la membrane 203 vers l'intérieur de la chambre 200. L'extrémité de la spatule 100 vient appuyer sur la membrane. En appuyant sur la membrane pour l'étirer vers le fond de la chambre 200, l'extrémité de la spatule atteint la surface d'appui abrasive 202 et vient broyer les espèces biologiques contre ladite surface afin de libérer les molécules d'ADN ;
- Au moins une unité de chauffage 12 du réseau de chambres fluidiques, qui est intégrée à la platine 14 et localisée par rapport à la zone de la cartouche 2 portant le réseau de chambres. L'unité de chauffage 12 peut comporter un élément Peltier, une plaque de cuivre pour homogénéiser la température, un capteur thermique permettant une régulation précise de la température, et éventuellement un radiateur et un ventilateur pour dissiper la chaleur ;
- Un système pneumatique 11 comportant une ou plusieurs pompes d'aspiration pour faire circuler l'échantillon fluidique dans chaque module fluidique MF1, MF2 de la cartouche et entre les deux modules. Le système pneumatique 11 peut être employé également pour générer un flux d'air (force d'aspiration Fvac_1, Fvac_2, Fvac_3) à travers le filtre 201 du dispositif de concentration et de lyse, notamment pour le faire sécher. Le système pneumatique peut aussi être configuré pour commander chaque vanne (de type pneumatique) employée dans les deux modules fluidiques MF1, MF2 ;
- Un système optique de mesure de fluorescence 13 agencé au-dessus de la platine 14, pouvant comporter une caméra, des diodes électroluminescentes et des filtres optiques, et configuré pour mesurer la fluorescence dans chacune des chambres du réseau de la cartouche, durant une réaction d'amplification d'ADN ;
- Une interface homme-machine HMI pour commander l'appareil d'analyse 1 ;
- Une unité de commande et de traitement UC pouvant comporter au moins un microprocesseur et des moyens de mémorisation. L'unité de commande et de traitement UC est destinée à exécuter une séquence de commande. Selon le type d'analyse à effectuer et selon les espèces biologiques à cibler, l'unité de commande et de traitement UC est configurée pour sélectionner la séquence de commande adaptée. Des unités déportées auprès de chaque entité matérielle du système pourront également être employées. L'unité de commande et de traitement UC est également à même de traiter les images capturées par le système optique de mesure 13. A partir de ces images, il est ainsi possible de remonter à la quantité d'ADN de l'échantillon présent dans chaque chambre du réseau et donc au nombre de bactéries initialement présentes dans l'échantillon initial prélevé sur site. L'unité de commande et de traitement UC est également configurée pour stocker les résultats dans une base de données DB stockée en local ou à distance et pour réaliser un suivi dans le temps en comparant les données à l'aide de différents filtres de sélection (lieu d'échantillonnage, type d'échantillon, cible,...).

Selon un aspect particulier de l'invention illustré par la figure 2, l'unité de commande et de traitement UC sera en mesure d'exécuter plusieurs modules logiciels M1, M2, M3 destinés chacun à la mise en œuvre de l'une des étapes E1, E2, E3 du processus d'analyse décrit ci-dessus. Chacun de ses modules logiciels aura pour fonction d'envoyer des commandes aux différentes entités de l'appareil et/ou de traiter des données reçues. En référence à la figure 2, on a ainsi le schéma suivant :
- Le module M1, destiné à la mise en œuvre de l'étape E1 de préparation, incluant la concentration et la lyse, est configuré pour envoyer des commandes au dispositif de concentration et de lyse 10 ainsi qu'au système pneumatique 11.
- Le module M2 destiné à la mise en œuvre de l'étape E2 de détection est configuré pour envoyer des commandes à l'unité de chauffage 12, au système pneumatique 11 ainsi qu'au système optique de mesure 13.
- Le module M3 destiné à la mise en œuvre de l'étape E3 d'analyse est configuré pour recevoir des images en provenance du système optique de mesure 13 et de traiter les images. À partir des images, il génère des résultats R d'analyse. Il peut notamment utiliser la base de données DB.
- L'interface homme-machine HMI permet de configurer l'appareil d'analyse et de sélectionner les paramètres d'analyse.

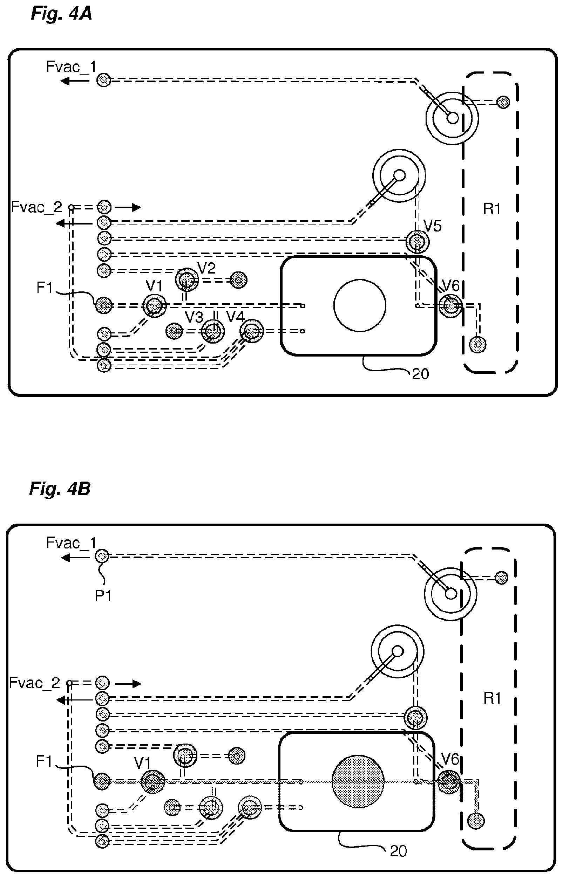

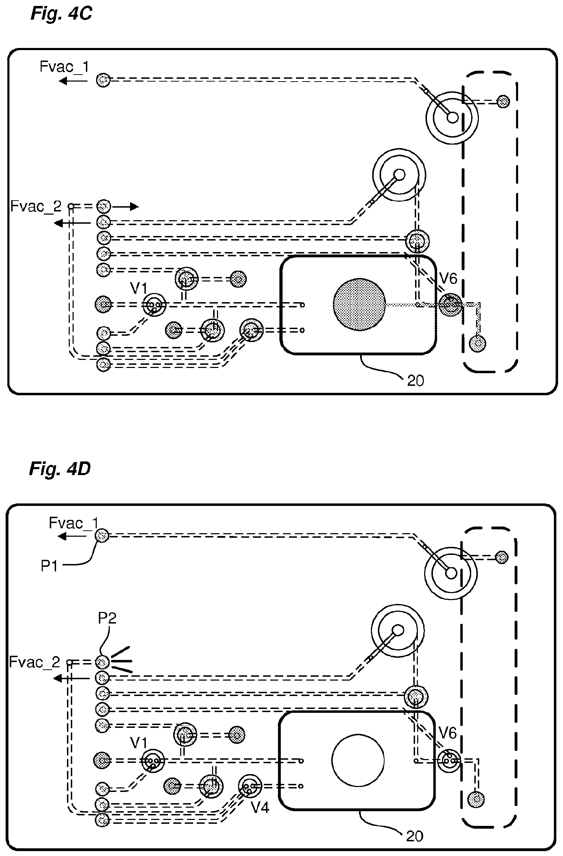

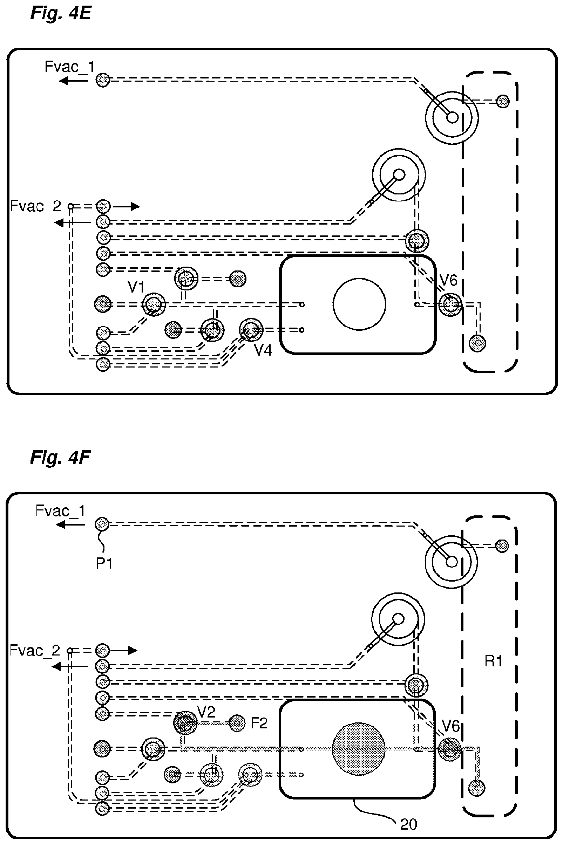

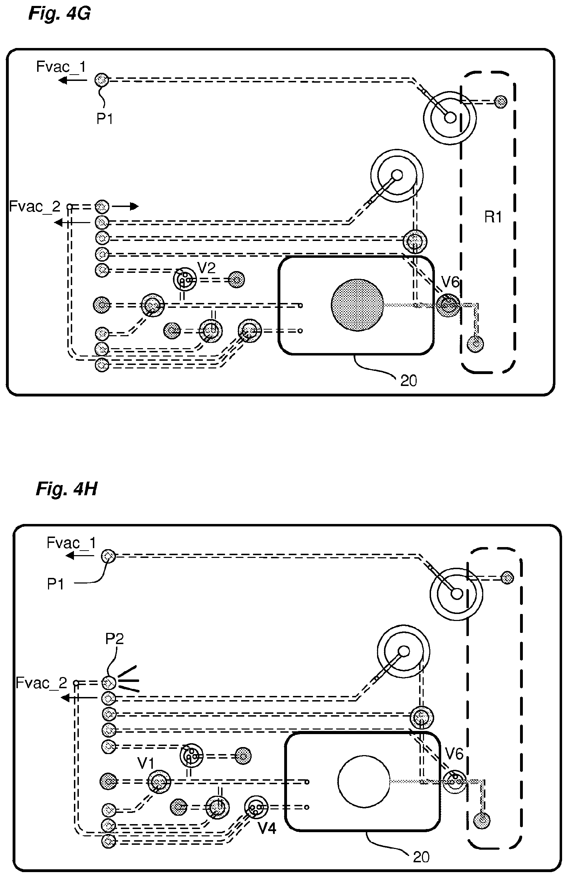

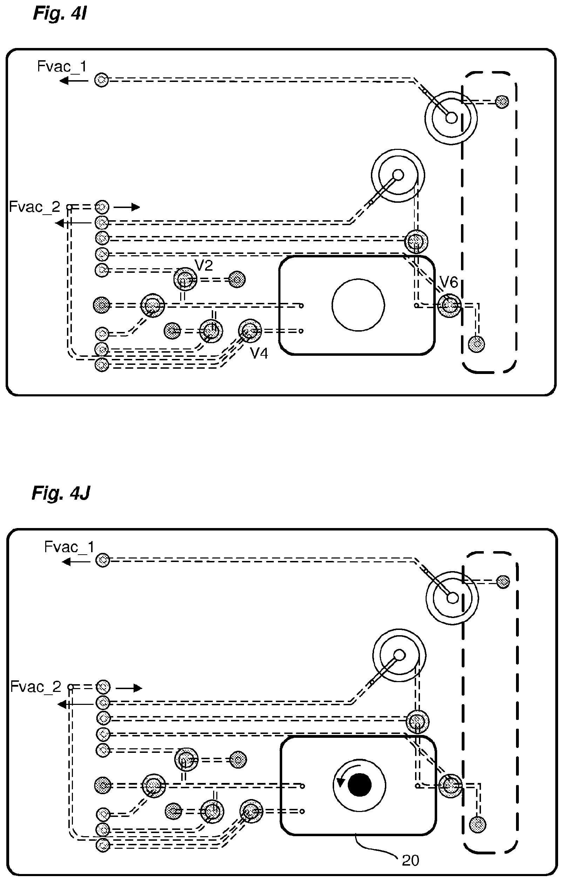

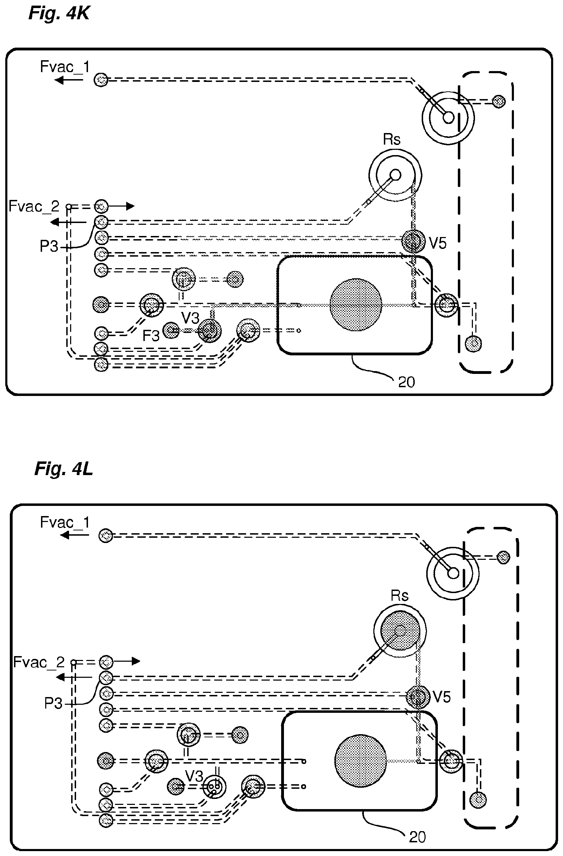

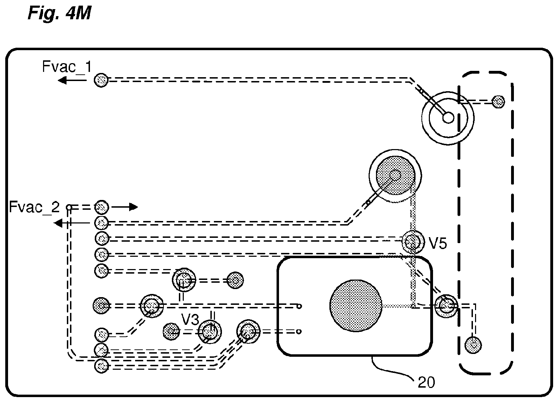

Les figures 4A à 4M illustrent en détails les différentes étapes mises en œuvre dans le premier module fluidique MF1 destiné à la concentration et à la lyse. Ces figures montrent le premier module fluidique MF1 réalisé sur une carte telle que décrite ci-dessus. De manière avantageuse, le module tel que représenté comporte ainsi :
- Plusieurs entrées/sorties pneumatiques Px destinées à être connectées au système pneumatique de l'appareil d'analyse lorsque la cartouche est insérée dans l'appareil ;
- Plusieurs entrées fluidiques Fy ;
- Le dispositif de concentration et de lyse 20 ;
- Un réservoir poubelle R1 ;
- Plusieurs vannes V1-V6 à deux voies, commandables par l'unité de commande et de traitement lors de l'exécution de la séquence ;
- Un réservoir de stockage Rs.

Dans ce module MF1, la circulation des différents fluides est effectuée en générant une force d'aspiration Fvac_1 ou Fvac_2 générée par le système pneumatique de l'appareil.

### Figure 4A

Le module est à l'état initial. Les vannes V1 à V6 sont toutes fermées.

### Figure 4B

Il s'agit de l'étape d'injection de l'échantillon prélevé dans le module. L'échantillon peut être injecté avec un volume de 1ml.

L'unité de commande et de traitement UC commande l'ouverture de la vanne V1 et l'ouverture de la vanne V6. L'unité de commande et de traitement UC commande le système pneumatique 11 pour générer à travers l'entrée P1 une force d'aspiration Fvac_1 afin d'aspirer l'échantillon dans le circuit fluidique. L'échantillon pénètre dans le circuit par l'entrée fluidique F1 puis rejoint la chambre 200 du dispositif de concentration et de lyse 20 et passe à travers le filtre 201 du dispositif 20. La partie de l'échantillon non retenue par le filtre 201 est évacuée vers le réservoir poubelle R1. La force d'aspiration Fvac_1 est maintenue tant que tout le volume d'échantillon n'a pas traversé le filtre.

### Figure 4C

En circulant à travers le filtre 201, l'échantillon est filtré par le filtre 201. Le surplus d'échantillon est évacué vers le réservoir poubelle R1.

### Figure 4D

Tout l'échantillon est filtré. Les espèces biologiques présentes dans l'échantillon et retenues par le filtre sont présentes dans la chambre.

L'unité de commande et de traitement UC commande l'ouverture de la vanne V4 et le système pneumatique 11. Une force d'aspiration Fvac_1 est générée par le point P1 et crée un flux d'air à travers le filtre 201 afin de sécher celui-ci. L'air est aspiré par le point P2.

### Figure 4E

L'unité de commande et de traitement commande la fermeture des vannes V1, V4 et V6. Toutes les vannes V1 à V6 sont alors fermées.

### Figure 4F

L'unité de commande et de traitement UC commande l'ouverture de la vanne V2 et l'ouverture de la vanne V6. L'unité de commande et de traitement UC commande le système pneumatique 11 pour générer à travers le point P1 la force d'aspiration Fvac_1 afin d'aspirer le liquide de rinçage par le point fluidique F2 et l'injecter dans la chambre 200 pour purifier les espèces biologiques filtrées. Le liquide de rinçage est évacué dans le réservoir poubelle R1.

### Figure 4G

Le rinçage est maintenu. Le liquide de rinçage peut être apporté dans un volume de 1ml.

### Figure 4H

L'unité de commande et de traitement commande l'ouverture de la vanne V4 et le système pneumatique 11. Une force d'aspiration Fvac_1 est générée par le point P1 et crée un flux d'air à travers le filtre 201 afin de sécher celui-ci. L'air est aspiré par le point P2.

### Figure 4I

L'unité de commande et de traitement commande la fermeture des vannes V2, V4 et V6. Toutes les vannes V1 à V6 sont fermées.

### Figure 4J

L'unité de commande et de traitement UC commande l'ensemble mécanique de lyse 10 afin de venir déplacer la spatule 100 dans un mouvement de broyage des espèces biologiques présentes dans la chambre 200 contre la surface d'appui abrasive 202. Le mouvement appliqué peut être la combinaison d'une rotation et de translations.

À l'issue de cette étape de lyse, la chambre 200 contient des polluants et un matériel biologique (des molécules d'ADN) à analyser.

### Figure 4K

L'unité de commande et de traitement UC commande l'ouverture de la vanne V3 et l'ouverture de la vanne V5.

L'unité de commande et de traitement UC commande le système pneumatique afin de générer la force d'aspiration Fvac_2 à travers le point P3 afin d'aspirer un liquide d'élution par le point fluidique F3 vers la chambre 200 et à travers le filtre 201 pour éluer le matériel biologique obtenu après la lyse.

Le liquide d'élution contenant le matériel biologique ciblé est recueilli dans un réservoir de stockage Rs. Ce liquide d'élution peut être injecté à un volume de 50µl.

### Figure 4L

Le liquide d'élution contenant le matériel biologique ciblé est recueilli dans le réservoir de stockage Rs.

### Figure 4M

L'unité de commande et de traitement commande la fermeture des vannes V3 et V5. Le processus de concentration et de lyse est alors terminé.

Dans ce premier module fluidique MF1, il faut noter que des filtres hydrophobes sont placés dans le circuit pour qu'aucun liquide ne vienne pénétrer l'appareil. Ces filtres sont intégrés à la cartouche, entre les entrées/sorties pneumatiques et le reste du circuit fluidique. Par ailleurs, ils permettent également de mieux ajuster le remplissage des volumes par aspiration. Ces filtres permettent en effet de laisser passer les gaz nécessaires à l'aspiration du liquide dans le module fluidique mais ne laissent pas passer les liquides.

Une fois le matériel biologique d'intérêt recueilli dans le réservoir de stockage Rs, celui-ci peut être transféré ou injecté dans le deuxième module fluidique MF2 de la cartouche pour détection.

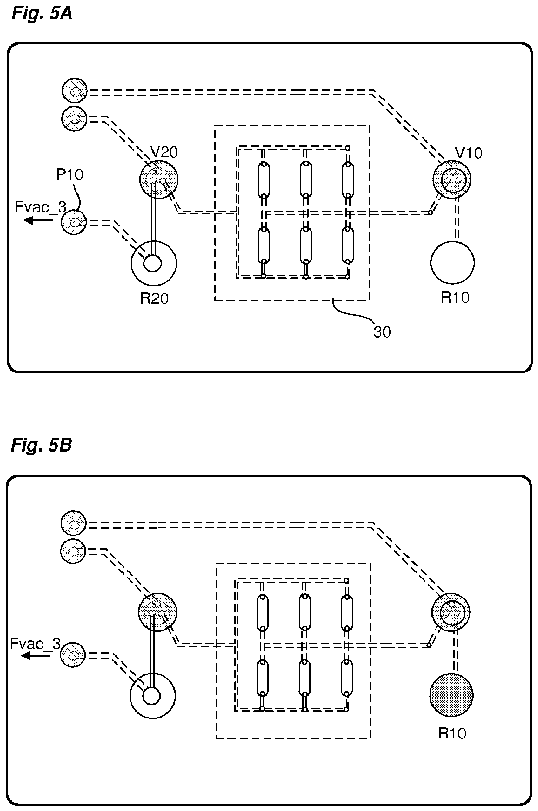

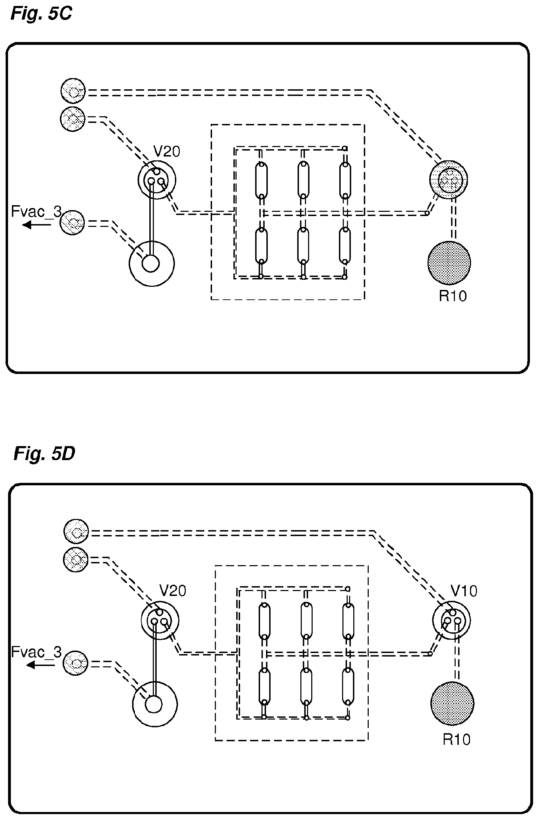

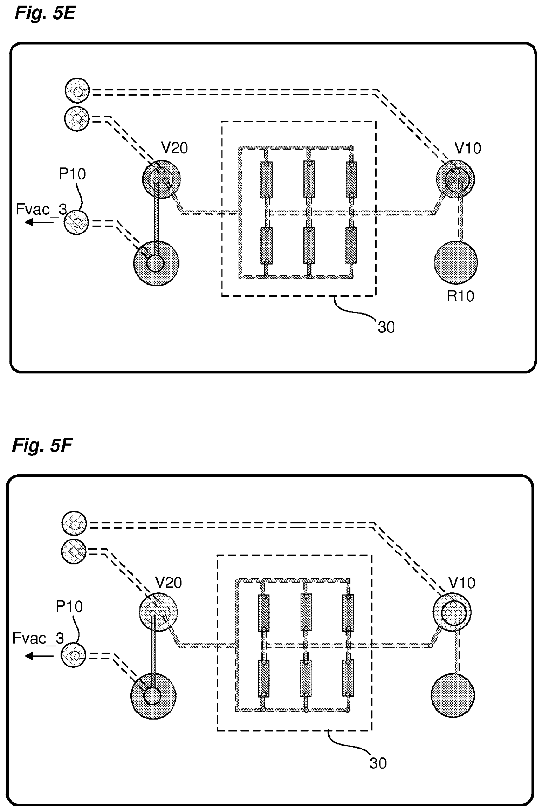

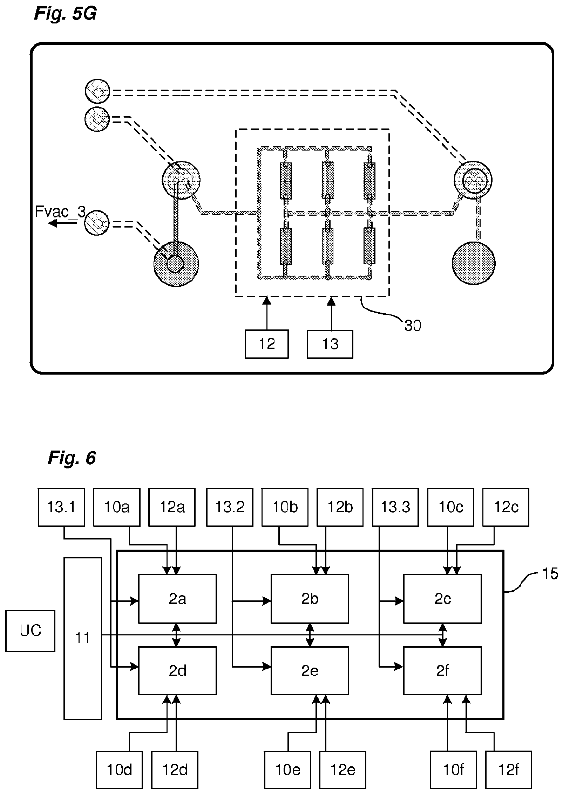

Les figures 5A à 5G illustrent en détails les différentes étapes mises en œuvre dans ce deuxième module fluidique MF2 destiné à la détection du matériel biologique récupéré à partir du prélèvement initial. Ces figures montrent le deuxième module fluidique MF2 réalisé sur une carte telle que décrite ci-dessus. De manière avantageuse, il faut noter que les deux modules MF1, MF2 peuvent être réalisés sur une même carte. De manière avantageuse, le module MF2 tel que représenté comporte ainsi :
- Plusieurs entrées/sorties pneumatiques Px destinées à être connectées au système pneumatique 11 de l'appareil d'analyse 1 lorsque la cartouche 2 est insérée dans l'appareil ;
- Un réservoir d'entrée R10 dans lequel est placé l'échantillon à analyser, ce réservoir pouvant être identique au réservoir de stockage Rs de fin de processus utilisé dans le premier module ;
- Un réservoir de sortie R20 ;
- Un réseau 30 de six chambres de détection alimentées en parallèle par un canal central de répartition ;
- Plusieurs vannes V10, V20 trois voies commandables par l'unité de commande et de traitement UC lors de l'exécution de la séquence d'analyse.

Dans ce module, la circulation des différents fluides est effectuée en générant une force d'aspiration Fvac_3 générée par le système pneumatique 11 de l'appareil à travers l'entrée P10.

### Figure 5A

Le module est à l'état initial. Les vannes V10 et V20 sont toutes deux fermées.

### Figure 5B

L'échantillon contenant le matériel biologique à analyser est placé dans le réservoir d'entrée R10. Ce réservoir peut être commun au réservoir de stockage Rs du premier module fluidique MF1.

### Figure 5C

L'unité de commande et de traitement UC commande l'ouverture de la vanne V20.

### Figure 5D

L'unité de commande et de traitement commande l'ouverture de la vanne V10.

### Figure 5E

L'unité de commande et de traitement UC commande le système pneumatique 11 pour générer une force d'aspiration Fvac_3 à travers le point P10 afin d'aspirer l'échantillon dans le circuit fluidique en dehors du réservoir R10. L'échantillon pénètre dans le canal central de répartition et, de manière simultanée, dans les six chambres en parallèle du réseau 30. L'architecture du réseau 30 de chambres permet de faire en sorte que les chambres soient toutes remplies en même temps.

### Figure 5F

L'unité de commande et de traitement UC commande la fermeture de la vanne V10 et la fermeture de la vanne V20 une fois que toutes les chambres du réseau sont remplies.

Chaque chambre peut comporter un réactif d'amplification distinct afin de détecter la présence de différentes espèces biologiques.

### Figure 5G

L'unité de commande et de traitement UC peut ensuite commander l'unité de chauffage 12 de l'appareil afin de faire subir au matériel biologique présent dans chaque chambre du réseau 30 un ou plusieurs cycles thermiques.

L'unité de commande et de traitement UC commande également le système optique de mesure 13 afin de capturer des images de fluorescence dans chaque chambre du réseau 30.

Une fois les images capturées, celles-ci sont enregistrées puis analysées par l'unité de commande et de traitement UC afin de déterminer quelles sont les espèces biologiques présentes et en quelles quantités celles-ci sont présentes. L'unité de commande et de traitement UC peut également se reporter aux analyses faites antérieurement afin de faire ressortir des tendances à la hausse ou à la baisse. Des résultats R sont ensuite générés par l'unité de commande et de traitement UC.

De manière avantageuse, les modules fluidiques MF1, MF2 peuvent employer des filtres hydrophobes afin de permettre des remplissages en mode aspiration avec un volume exact.

Dans le deuxième module fluidique MF2, un volume mort peut être intégré dans le canal central de répartition afin de piéger les bulles d'air générées lors du remplissage des chambres du réseau 30. De même, chaque chambre du réseau 30 peut avoir une hauteur limitée afin d'éviter la formation de bulles à l'entrée.

De manière avantageuse, chaque vanne des modules fluidiques peut employer un joint réalisé en EPDM, ceci afin d'améliorer l'étanchéité des circuits et d'éviter l'injection d'air dans les circuits lorsque la vanne est fermée.

Selon un aspect particulier de l'invention illustré par la figure 6, l'appareil peut être adapté pour traiter plusieurs cartouches de manière simultanée, par exemple six cartouches 2a, 2b, 2c, 2d, 2e, 2f. Celles-ci seront chargées sur une cassette 15 positionnée sur la platine 14 de l'appareil qui contient ainsi un système pneumatique 11 commun à toutes les cartouches avec un nombre d'entrées/sorties adaptées, six ensembles mécaniques 10a, 10b, 10c, 10d, 10e, 10f actionnables pour effectuer la lyse dans chaque cartouche, six zones chauffantes 12a, 12b, 12c, 12d, 12e, 12f et trois caméras 13.1, 13.2, 13.3 qui se déplacent chacune entre deux positions, afin d'imager les six cartouches. L'unité de commande et de traitement UC permet bien entendu de commander les différentes entités et de recueillir les images capturées pour les traiter.

Ainsi le système de préparation, de détection et d'analyse de l'invention présente les avantages suivants :
▪ Il est facilement transportable dans un environnement industriel ;
▪ Il permet une analyse rapide d'un échantillon (pas plus de quelques heures) ;
▪ Il est simple d'utilisation car il permet de passer de l'échantillon au résultat de façon automatisé, en limitant au maximum l'intervention d'un opérateur ;
▪ Il est compatible avec des échantillons complexes par exemple à forte salinité, contenant par exemple des solides dissous, hydrocarbures libres ou dissous, cations en fortes concentration, pH acides issus d'installations industrielles ;
▪ Il peut s'adapter aux cibles à détecter, pour un groupe taxonomique particulier ou pour une fonction métabolique d'intérêt (par exemple les bactéries sulfato-réductrices), et permet grâce à sa structure l'analyse simultanée de plusieurs espèces/fonctions ou groupe d'espèces/fonctions ;
▪ Il permet d'obtenir des résultats qualitatifs et quantitatifs (grâce à la Q-PCR avec calibration) ;
▪ Il est sensible, sa limite de détection étant inférieure à 10³ cfu/mL ;
▪ Il permet, grâce à une base de données, de mettre à disposition des données d'évolution quantitatives et qualitatives des espèces biologiques détectés au cours du temps.

## Revendications

1. Système automatisé de préparation, de détection et d'analyse d'un premier échantillon fluidique contenant des espèces biologiques, ledit système de préparation, de détection et d'analyse comprenant :
- Au moins une cartouche fluidique (2) qui comporte au moins un module fluidique (MF1) de concentration et de lyse et un module fluidique (MF2) de détection comprenant un réseau (30) de plusieurs chambres d'amplification agencées en parallèle par rapport à un canal fluidique de répartition, ledit module fluidique (MF1) de concentration et de lyse comprenant un dispositif de concentration et de lyse (20) qui comporte une chambre de préparation (200), une surface d'appui abrasive (202) réalisée dans ladite chambre de préparation, un filtre (201) et une membrane (203) souple et déformable venant refermer ladite chambre,
- Un appareil comprenant :
o Un châssis doté d'au moins une platine (14) destinée à recevoir ladite cartouche (2) fluidique de manière amovible,
o Un ensemble mécanique comprenant au moins une tige (100) mobile fixée audit châssis et comprenant une extrémité libre agencée pour coopérer avec ladite membrane (203) souple du module fluidique (MF1) de concentration et de lyse,
o Un système pneumatique (11) commandé pour faire circuler un fluide à travers ladite cartouche,
o Au moins une unité de chauffage (12) portée par la platine et agencée pour chauffer le réseau de chambres en parallèle lors d'une réaction d'amplification, cyclique ou isotherme,
o Un système optique de mesure (13) de fluorescence à travers une ou plusieurs des chambres d'amplification du module fluidique (MF2) de détection de la cartouche,
o Une unité de commande et de traitement (UC) configurée pour mettre en œuvre une séquence d'analyse qui comporte au moins les étapes suivantes :
▪ Commande du système pneumatique (11) pour injection du premier échantillon fluidique à travers le filtre (201) du dispositif de concentration et de lyse pour récupérer les espèces biologiques présentes dans le premier échantillon,
▪ Commande du système pneumatique pour générer un flux d'air de séchage dans un circuit fluidique du module fluidique (MF1) de concentration et de lyse, ledit circuit fluidique traversant ledit filtre du dispositif de concentration et de lyse (20),
▪ Commande de l'ensemble mécanique (10) pour actionnement de la tige dans un mouvement d'appui de la membrane souple contre la surface d'appui abrasive afin de lyser les espèces biologiques contenues dans le premier échantillon fluidique,
▪ Commande du système pneumatique (11) pour évacuer un deuxième échantillon fluidique en dehors de la chambre de préparation vers ledit canal fluidique de répartition de la cartouche,
▪ Commande du système pneumatique (11) pour remplissage en parallèle et de manière simultanée des chambres d'amplification du réseau (30) avec le deuxième échantillon fluidique,
▪ Commande de l'unité de chauffage (12) pour chauffer ledit deuxième échantillon fluidique présent dans chaque chambre du réseau (30),
▪ Commande du système optique de mesure (13) pour mesurer la fluorescence dans chaque chambre d'amplification contenant une fraction du deuxième échantillon fluidique,
▪ Enregistrement des résultats de mesure de fluorescence,
▪ Application d'un algorithme d'analyse sur les résultats de mesure obtenus et génération de données qualitatives et quantitatives des espèces biologiques présentes dans le premier échantillon fluidique.

2. Système selon la revendication 1, **caractérisé en ce que** l'unité de commande et de traitement est configurée pour exécuter :
- Un premier module (M1) configuré pour envoyer des commandes au dispositif de concentration et de lyse (10) ainsi qu'au système pneumatique (11),
- Un deuxième module (M2) configuré pour envoyer des commandes à l'unité de chauffage (12), au système pneumatique (11) ainsi qu'au système optique de mesure (13),
- Un troisième module (M3) configuré pour recevoir des images en provenance du système optique de mesure (13) et de traiter les images.

3. Système selon la revendication 2, **caractérisé en ce qu'**il comporte une base de données stockant les résultats d'analyse antérieures et **en ce que** le troisième module est configuré pour se reporter à ladite base de données afin de générer des courbes de variation de concentration de chaque espèce biologique détecté.

4. Système selon l'une des revendications 1 à 3, **caractérisé en ce que** chaque module fluidique comporte des filtres hydrophobes agencés sur des canaux pneumatiques reliés directement au système pneumatique.

5. Système selon l'une des revendications 1 à 4, **caractérisé en ce que** le module fluidique (MF1) de concentration et de lyse et le module fluidique (MF2) de détection comporte plusieurs vannes fluidiques commandables par l'unité de commande et de traitement (UC).

6. Système selon l'une des revendications 1 à 5, **caractérisé en ce qu'**il comporte une cassette (15) intégrant n cartouches fluidiques, avec n supérieur ou égal à 2.

7. Système selon la revendication 6, **caractérisé en ce qu'**il comporte :
- n ensembles mécaniques comprenant chacun au moins une tige (100) et associée à chaque cartouche,
- n unités de chauffage (12) agencés chacune pour chauffer le réseau de chambres de chaque cartouche,
- n/2 système optique de mesure (13) de fluorescence, configuré pour se déplacer entre deux positions, afin d'imager les n cartouches du système.

8. Procédé de préparation, de détection et d'analyse d'un échantillon fluidique, mis en œuvre à partir du système tel que défini dans l'une des revendications précédentes, **caractérisé en ce qu'**il comporte les étapes suivantes :
- Commande du système pneumatique (11) pour injection du premier échantillon fluidique à travers le filtre (201) du dispositif de concentration et de lyse pour récupérer les espèces biologiques présentes dans le premier échantillon,
- Commande du système pneumatique pour générer un flux d'air de séchage dans un circuit fluidique du module fluidique (MF1) de concentration et de lyse, ledit circuit fluidique traversant ledit filtre du dispositif de concentration et de lyse (20),
- Commande de l'ensemble mécanique (10) pour actionnement de la tige dans un mouvement d'appui de la membrane souple contre la surface d'appui abrasive afin de lyser les espèces biologiques contenues dans le premier échantillon fluidique,
- Commande du système pneumatique (11) pour évacuer un deuxième échantillon fluidique en dehors de la chambre de préparation vers ledit canal fluidique de répartition de la cartouche,
- Commande du système pneumatique (11) pour remplissage en parallèle et de manière simultanée des chambres d'amplification du réseau (30) avec le deuxième échantillon fluidique,
- Commande de l'unité de chauffage (12) pour chauffer ledit deuxième échantillon fluidique présent dans chaque chambre du réseau (30),
- Commande du système optique de mesure (13) pour mesurer la fluorescence dans chaque chambre d'amplification contenant une fraction du deuxième échantillon fluidique,
- Enregistrement des résultats de mesure de fluorescence,
- Application d'un algorithme d'analyse sur les résultats de mesure obtenus et génération de données qualitatives et quantitatives des espèces biologiques présentes dans le premier échantillon fluidique.

9. Utilisation du système tel que défini dans l'une des revendications 1 à 7 pour détecter la présence d'espèces biologiques dans un échantillon fluidique dans ladite cartouche fluidique du système.

## Patentansprüche

1. Automatisiertes System zur Vorbereitung, Detektion und Analyse einer ersten Fluidprobe, die biologische Spezies enthält, wobei das Vorbereitungs-, Detektions- und Analysesystem umfasst:
- mindestens eine Fluidkartusche (2), die mindestens ein Konzentrations- und Lysefluidmodul (MF1) und ein Detektionsfluidmodul (MF2) aufweist, umfassend ein Netz (30) aus mehreren Amplifikationskammern, die in Bezug auf einen Verteilungsfluidkanal parallel eingerichtet sind, wobei das Konzentrations- und Lysefluidmodul (MF1) eine Konzentrations- und Lysevorrichtung (20) umfasst, die eine Vorbereitungskammer (200), eine in der Vorbereitungskammer ausgeführte abrasive Anlagefläche (202), einen Filter (201) und eine elastische und verformbare Membran (203), welche die Kammer verschließt, aufweist,
- ein Gerät, umfassend:
o einen Rahmen, der mit mindestens einer Platte (14) versehen ist, die dazu bestimmt ist, die Fluidkartusche (2) lösbar aufzunehmen,
o eine mechanische Anordnung, die mindestens einen beweglichen Stab (100) umfasst, der an dem Rahmen befestigt ist und ein freies Ende umfasst, das dazu eingerichtet ist, mit der elastischen Membran (203) des Konzentrations- und Lysefluidmoduls (MF1) zusammenzuwirken,
o ein pneumatisches System (11), das gesteuert wird, um ein Fluid durch die Kartusche hindurch zirkulieren zu lassen,
o mindestens eine Heizeinheit (12), die von der Platte getragen wird und dazu eingerichtet ist, das Netz aus parallelen Kammern bei einer zyklischen oder isothermen Amplifikationsreaktion zu erhitzen,
o ein optisches Messsystem (13) zur Messung von Fluoreszenz durch eine oder mehrere der Amplifikationskammern des Detektionsfluidmoduls (MF2) der Kartusche hindurch,
o eine Steuer- und Verarbeitungseinheit (UC), die dazu ausgestaltet ist, eine Analysesequenz umzusetzen, die mindestens die folgenden Schritte aufweist:
▪ Steuern des pneumatischen Systems (11) zum Injizieren der ersten Fluidprobe durch den Filter (201) der Konzentrations- und Lysevorrichtung hindurch, um die in der ersten Probe vorhandenen biologischen Spezies zurückzugewinnen,
▪ Steuern des pneumatischen Systems, um einen Trocknungsluftstrom in einem Fluidkreislauf des Konzentrations- und Lysefluidmoduls (MF1) zu erzeugen, wobei der Fluidkreislauf den Filter der Konzentrations- und Lysevorrichtung (20) durchquert,
▪ Steuern der mechanischen Anordnung (10) zum Betätigen des Stabs in eine Bewegung zur Anlage der elastischen Membran an die abrasive Anlagefläche, um die in der ersten Fluidprobe enthaltenen biologischen Spezies zu lysieren,
▪ Steuern des pneumatischen Systems (11), um eine zweite Fluidprobe aus der Vorbereitungskammer zum Verteilungsfluidkanal der Kartusche hin wegzuführen,
▪ Steuern des pneumatischen Systems (11) zum parallelen und gleichzeitigen Füllen der Amplifikationskammern des Netzes (30) mit der zweiten Fluidprobe,
▪ Steuern der Heizeinheit (12), um die in jeder Kammer des Netzes (30) vorhandene zweite Fluidprobe zu erhitzen,
▪ Steuern des optischen Messsystems (13), um die Fluoreszenz in jeder Amplifikationskammer, die eine Fraktion der zweiten Fluidprobe enthält, zu messen,
▪ Aufzeichnen der Fluoreszenzmessergebnisse,
▪ Anwenden eines Analysealgorithmus auf die erhaltenen Messergebnisse und Erzeugen von qualitativen und quantitativen Daten der in der ersten Fluidprobe vorhandenen biologischen Spezies.

2. System nach Anspruch 1, **dadurch gekennzeichnet, dass** die Steuer- und Verarbeitungseinheit dazu ausgestaltet ist, abzuarbeiten:
- ein erstes Modul (M1), das dazu ausgestaltet ist, Befehle an die Konzentrations- und Lysevorrichtung (10) sowie an das pneumatische System (11) zu senden,
- ein zweites Modul (M2), das dazu ausgestaltet ist, Befehle an die Heizeinheit (12), an das pneumatische System (11) sowie an das optische Messsystem (13) zu senden,
- ein drittes Modul (M3), das dazu ausgestaltet ist, vom optischen Messsystem (13) kommende Bilder zu empfangen und die Bilder zu verarbeiten.

3. System nach Anspruch 2, **dadurch gekennzeichnet, dass** es eine Datenbank aufweist, welche die früheren Analyseergebnisse speichert, und dass das dritte Modul dazu ausgestaltet ist, sich auf die Datenbank zu beziehen, um Konzentrationsänderungskurven jeder detektierten biologischen Spezies zu erzeugen.

4. System nach einem der Ansprüche 1 bis 3, **dadurch gekennzeichnet, dass** jedes Fluidmodul hydrophobe Filter aufweist, die auf pneumatischen Kanälen eingerichtet sind, die direkt mit dem pneumatischen System verbunden sind.

5. System nach einem der Ansprüche 1 bis 4, **dadurch gekennzeichnet, dass** das Konzentrations- und Lysefluidmodul (MF1) und das Detektionsfluidmodul (MF2) mehrere Fluidventile umfassen, die von der Steuer- und Verarbeitungseinheit (UC) steuerbar sind.

6. System nach einem der Ansprüche 1 bis 5, **dadurch gekennzeichnet, dass** es eine Kassette (15) aufweist, die n Fluidkartuschen mit n größer oder gleich 2 aufweist.

7. System nach Anspruch 6, **dadurch gekennzeichnet, dass** es aufweist:
- n mechanische Anordnungen, die jeweils mindestens einen Stab (100) umfassen, der jeder Kartusche zugeordnet ist,
- n Heizeinheiten (12), die jeweils dazu eingerichtet sind, das Netz aus Kammern jeder Kartusche zu erhitzen,
- n/2 optische Fluoreszenzmesssysteme (13), die dazu ausgestaltet sind, sich zwischen zwei Positionen zu verlagern, um die n Kartuschen des Systems abzubilden.

8. Verfahren zur Vorbereitung, Detektion und Analyse einer Fluidprobe, das ausgehend von dem wie in einem der vorhergehenden Ansprüche definierten System umgesetzt wird, **dadurch gekennzeichnet, dass** es die folgenden Schritte aufweist:
- Steuern des pneumatischen Systems (11) zum Injizieren der ersten Fluidprobe durch den Filter (201) der Konzentrations- und Lysevorrichtung hindurch, um die in der ersten Probe vorhandenen biologischen Spezies zurückzugewinnen,
- Steuern des pneumatischen Systems, um einen Trocknungsluftstrom in einem Fluidkreislauf des Konzentrations- und Lysefluidmoduls (MF1) zu erzeugen, wobei der Fluidkreislauf das Filter der Konzentrations- und Lysevorrichtung (20) durchquert,
- Steuern der mechanischen Anordnung (10) zum Betätigen des Stabs in eine Bewegung zur Anlage der elastischen Membran an die abrasive Anlagefläche, um die in der ersten Fluidprobe enthaltenen biologischen Spezies zu lysieren,
- Steuern des pneumatischen Systems (11), um eine zweite Fluidprobe aus der Vorbereitungskammer zum Verteilungsfluidkanal der Kartusche hin wegzuführen,
- Steuern des pneumatischen Systems (11) zum parallelen und gleichzeitigen Füllen der Amplifikationskammern des Netzes (30) mit der zweiten Fluidprobe,
- Steuern der Heizeinheit (12), um die in jeder Kammer des Netzes (30) vorhandene zweite Fluidprobe zu erhitzen,
- Steuern des optischen Messsystems (13), um die Fluoreszenz in jeder Amplifikationskammer, die eine Fraktion der zweiten Fluidprobe enthält, zu messen,
- Aufzeichnen der Fluoreszenzmessergebnisse,
- Anwenden eines Analysealgorithmus auf die erhaltenen Messergebnisse und Erzeugen von qualitativen und quantitativen Daten der in der ersten Fluidprobe vorhandenen biologischen Spezies.

9. Verwenden des wie in einem der Ansprüche 1 bis 7 definierten Systems, um das Vorkommen von biologischen Spezies in einer Fluidprobe in der Fluidkartusche des Systems zu detektieren.

## Claims

1. Automated preparation, detection and analysis system for a first fluid sample containing biological species, said preparation, detection and analysis system including:
- at least one fluid cartridge (2) which includes at least one fluid concentration and lysis module (MF1) and a fluid detection module (MF2) comprising a network (30) of a plurality of amplification chambers which are arranged in parallel relative to a fluid distribution channel, said fluid concentration and lysis module (MF1) including a concentration and lysis device (20) which includes a preparation chamber (200), an abrasive abutment surface (202) which is produced in said preparation chamber, a filter (201) and a flexible and deformable membrane (203) which closes said chamber,
- a device including:
o a frame which is provided with at least one plate (14) which is intended to receive said fluid cartridge (2) in a removable manner,
o a mechanical assembly including at least one movable rod (100) which is fixed to said frame and which comprises a free end which is arranged to cooperate with said flexible membrane (203) of the fluid concentration and lysis module (MF1),
o a pneumatic system (11) controlled to enable circulation of a fluid through said cartridge,
o at least one heating unit (12) which is carried by the plate and which is arranged in order to heat the network of chambers in parallel during a cyclical or isothermal amplification reaction,
o an optical system (13) for measuring fluorescence through one or more of the amplification chambers of the fluid detection module (MF2) of the cartridge,
o a control and processing unit (UC) which is configured to implement an analysis sequence which comprises at least the following steps:
▪ controlling the pneumatic system (11) in order to inject the first fluid sample through the filter (201) of the concentration and lysis device in order to recover the biological species present in the first sample,
▪ controlling the pneumatic system in order to generate a flow of drying air in a fluid circuit of the fluid concentration and lysis module (MF1), said fluid circuit passing through said filter of the concentration and lysis device (20),
▪ controlling the mechanical assembly (10) in order to activate the rod in an abutment movement of the flexible membrane against the abrasive abutment surface in order to lyse the biological species contained in the first fluid sample,
▪ controlling the pneumatic system (11) in order to discharge a second fluid sample out of the preparation chamber to said fluid distribution channel of the cartridge,
▪ controlling the pneumatic system (11) in order to fill in parallel and in a simultaneous manner the amplification chambers of the network (30) with the second fluid sample,
▪ controlling the heating unit (12) in order to heat said second fluid sample present in each chamber of the network (30),
▪ controlling the optical measurement system (13) in order to measure the fluorescence in each amplification chamber containing a fraction of the second fluid sample,
▪ recording the fluorescence measurement results,
▪ applying an analysis algorithm to the measurement results obtained and generating qualitative and quantitative data of the biological species present in the first fluid sample.

2. System according to Claim 1, **characterized in that** the control and processing unit is configured to carry out:
- a first module (M1) which is configured to send commands to the concentration and lysis device (10) and to the pneumatic system (11),
- a second module (M2) which is configured to send commands to the heating unit (12), to the pneumatic system (11) and to the optical measurement system (13),
- a third module (M3) which is configured to receive images from the optical measurement system (13) and to process the images.

3. System according to Claim 2, **characterized in that** it includes a database which stores the previous analysis results and **in that** the third module is configured to refer to said database in order to generate concentration variation curves for each biological species detected.

4. System according to one of Claims 1 to 3, **characterized in that** each fluid module includes hydrophobic filters which are arranged on pneumatic channels which are connected directly to the pneumatic system.

5. System according to one of Claims 1 to 4, **characterized in that** the fluid concentration and lysis module (MF1) and the fluid detection module (MF2) include a plurality of fluid valves which can be controlled by the control and processing unit (UC).

6. System according to one of Claims 1 to 5, **characterized in that** it comprises a cartridge (15) which integrates n fluid cartridges, with n being greater than or equal to 2.

7. System according to Claim 6, **characterized in that** it includes:
- n mechanical assemblies which each comprise at least one rod (100) which is associated with each cartridge,
- n heating units (12) which are each arranged in order to heat the network of chambers of each cartridge,
- n/2 optical fluorescence measurement system (13) which is configured to move between two positions in order to image the n cartridges of the system.

8. Preparation, detection and analysis method for a fluid sample, implemented using the system as defined in one of the preceding claims, **characterized in that** it includes the following steps:
- controlling the pneumatic system (11) in order to inject the first fluid sample through the filter (201) of the concentration and lysis device in order to recover the biological species present in the first sample,
- controlling the pneumatic system in order to generate a flow of drying air in a fluid circuit of the fluid concentration and lysis module (MF1), said fluid circuit passing through said filter of the concentration and lysis device (20),
- controlling the mechanical assembly (10) in order to activate the rod in a movement for abutting the flexible membrane against the abrasive abutment surface in order to lyse the biological species contained in the first fluid sample,
- controlling the pneumatic system (11) in order to discharge a second fluid sample out of the preparation chamber to said fluid distribution channel of the cartridge,
- controlling the pneumatic system (11) in order to fill in parallel and in a simultaneous manner the amplification chambers of the network (30) with the second fluid sample,
- controlling the heating unit (12) in order to heat said second fluid sample present in each chamber of the network (30),
- controlling the optical measurement system (13) in order to measure the fluorescence in each amplification chamber containing a fraction of the second fluid sample,
- recording the fluorescence measurement results,
- applying an analysis algorithm to the measurement results obtained and generating qualitative and quantitative data of the biological species present in the first fluid sample.

9. Use of the system as defined in one of Claims 1 to 7 in order to detect the presence of biological species in a fluid sample in said fluid cartridge of the system.
